# EUROPEAN PATENT SPECIFICATION

(11) **EP 1 543 031 B1**
(45) Date of publication and mention of the grant of the patent: **16.08.2006**
(21) Application number: 03765167.6
(22) Date of filing: 17.07.2003
(51) Int. Cl.: C07K 14/62, A61K 38/28

(54) **MONOMERIC INSULIN**
MONOMERES INSULIN
MONOMÈRE DE L'INSULINE

(30) Priority: 19.07.2002 CN 02136107
(43) Date of publication of application: 22.06.2005
(73) Proprietor: Genemedix PLC, Newmarket, Suffolk CB8 7XN (GB); Shanghai C.A.S. Shenglongda Biotech (Group) Company, Shanghai (CN)
(72) Inventor: ZHANG, Youshang, Shanghai 200031 (CN); DING, Jinguo, Shanghai 200031 (CN); SHI, Jaihao, Shanghai 200031 (CN); CUI, Dafu, Shanghai 200031 (CN)
(74) Representative: Hyden, Martin Douglas
(86) International application number: PCT/GB2003/003136
(87) International publication number: WO 2004/009629

(56) References cited:
- WO-A-96/29344
- US-A- 4 946 828
- YE ET AL: "Crystal structure of destripeptide (B28-B30) insulin: implications for insulin dissociation" BIOCHIMICA ET BIOPHYSICA ACTA , vol. 1547, no. 1, 2001, pages 18-25, XP004239369 ISSN: 0167-4838
- MARKUSSEN ET AL: "Soluble, prolonged-acting insulin derivatives. II. Degree of protraction and crystallizability of insulins substituted in positions A17, B8, B13, B27 and B30" PROTEIN ENGINEERING, vol. 1, 1987, pages 215-223, XP001018792 ISSN: 0269-2139
- DING ET AL: "Monomeric B27 Lys Destripeptide Insulin: Semisynthesis, characterization and biological activity " ACTA BIOCHEMICA ET BIOPHYSICA SINICA, vol. 35, no. 3, March 2003 (2003-03), pages 215-218, XP008024665

### Sequence Table

<110>GeneMedix plc; Shanghai C.A. S Shenglongda Biotech(Group) Company; ZHANG Youshang; DING Jinguo; CUID afu; SHI Jiahao
<120> A Novel Monomeric Insulin, a Pharmaceutical Composition comprising said monomeric insulin, and Preparation thereof
<130> GMD 00115/WO
<140> PCT/GB 03/03136
   < 141 > 2003 07 17
   <150> CN 02136107.X
   <151> 2003 07 19
   <160> 5
<170> Patent In version 3.1
<210>
   <211> 27
   <212> PRT
   <213> Artificial Sequence
<220>
   <221> MISC_FEATURE
   <222> (23)..(26)
   <223> Xaa=Gly, Ala, Asp, Glu, Asn, Gln, Ser, Thr, Leu, Ile, Lys, Arg, P he,Tyr, Trp, Pro, Cys, Met, His, or Val
<220>
   <221> MISC_FEATURE
   <222> (27)..(27)
   <223> Xaa=Lys or Arg
<220>
   <221> MISC_FEATURE
   <222> (1)..(27)
   <223> Insulin B Chain
<400> 1
<210> 2
   <211> 21
   <212> PRT
   <213> Artificial Sequence
<220>
   <221> MISC_FEATURE
   <222> (1)..(21)
   <223> Insulin A Chain
<400> 2
   Gly Ile Val Glu Gln Cys Cys Thr Ser Ile Cys Ser Leu Tyr Gln Leu
   1 5 10 15
   Glu Asn Tyr Cys Asn
   20
<210> 3
   <211> 153
   <212> DNA
   <213> Artificial Sequence
<220>
   <221> CDS
   <222> (1)..(153)
   <223>
<400> 3
   ttc gtt aac caa cac ttg tgc ggt tcc cac ttg gtt gag gct ttg tac 48
   Phe Val Asn Gln His Leu Cys Gly Ser His Leu Val Glu Ala Leu Tyr
   1 5 10 15
   ttg gtt tgc ggt gaa aga ggt ttc ttc tac aag gct gct aag ggt att 96
   Leu Val Cys Gly Glu Arg Gly Phe Phe Tyr Lys Ala Ala Lys Gly Ile
   20 25 30
   gtc gaa caa tgc tgt acc tcc atc tgc tcc ttg tac caa ttg gaa aac 144
   Val Glu Gln Cys Cys Thr Ser Ile Cys Ser Leu Tyr Gln Leu Glu Asn
   35 40 45
   tac tgc aac 153
<210> 4
   <211> 51
   <212> PRT
   <213> Artificial Sequence
<400> 4
   Phe Val Asn Gln His Leu Cys Gly Ser His Leu Val Glu Ala Leu Tyr
   1 5 10 15
   Leu Val Cys Gly Glu Arg Gly Phe Phe Tyr Lys Ala Ala Lys Gly Ile
   20 25 30
   Val Glu Gln Cys Cys Thr Ser Ile Cys Ser Leu Tyr Gln Leu Glu Asn
   35 40 45
   Tyr Cys Asn
   50
<210> 5
   <211> 27
   <212> PRT
   <213> Artificial Sequence
   <220>
   <221> MISC_FEATURE
   <222> (1)..(27)
   <223> Insulin B Chain B27K-DTrI
<400> 5

### Sequence Table

<110>GeneMedix plc; Shanghai C.A.S Shenglongda Biotech(Group) Company; ZHANG Youshang, DING Jinguo; CUID afu; SHI Jiahao
<120> A Novel Monomeric Insulin, a Pharmaceutical Composition comprising said monomeric insulin, and Preparation thereof
<130> GMD 00115/WO
   <140> PCT/GB 03/03136
   <141> 2003 07 17
   <150> CN 02136107.X
   < 151 > 2003 07 19
   <160> 5
< 170> Patent In version 3.1
<210> 1
   <211> 27
   <212> PRT
   <213> Artificial Sequence
   <220>
   <221> MISC_FEATURE
   <222> (23)..(26)
   <223> Xaa=Gly, Ala, Asp, Glu, Asn, Gln, Ser, Thr, Leu, Ile, Lys, Arg, Phe, Tyr, Trp, Pro, Cys, Met, His, or Val
<220>
   <221> MISC_FEATURE
   <222> (27)..(27)
   <223> Xaa=Lys or Arg
   <220>
   <221> MISC_FEATURE
   <222> (1)..(27)
   <223> Insulin B Chain
<400> 1
   Phe Val Asn Gln His Leu Cys Gly Ser His Leu Val Glu Ala Leu Tyr
   1 5 10 15
   Leu Val Cys Gly Glu Arg Xaa Xaa Xaa Xaa Xaa
   20 25
<210> 2
   <211> 21
   <212> PRT
   <213> Artificial Sequence
<220>
   <221> MISC_FEATURE
   <222> (1)..(21)
   <223> Insulin A Chain
<400> 2
   Gly Ile Val Glu Gln Cys Cys Thr Ser Ile Cys Ser Leu Tyr Gln Leu
   1 5 10 15
   Glu Asn Tyr Cys Asn
   20
<210> 3
   <211> 153
   <212> DNA
   <213> Artificial Sequence
<220>
   <221> CDS
   <222> (1)..(153)
   <223>
<400> 3
   ttc gtt aac caa cac ttg tgc ggt tcc cac ttg gtt gag gct ttg tac 48
   Phe Val Asn Gln His Leu Cys Gly Ser His Leu Val Glu Ala Leu Tyr
   1 5 10 15
   ttg gtt tgc ggt gaa aga ggt ttc ttc tac aag gct gct aag ggt att 96
   Leu Val Cys Gly Glu Arg Gly Phe Phe Tyr Lys Ala Ala Lys Gly Ile
   20 25 30
   gtc gaa caa tgc tgt acc tcc atc tgc tcc ttg tac caa ttg gaa aac 144
   Val Glu Gln Cys Cys Thr Ser Ile Cys Ser Leu Tyr Gln Leu Glu Asn
   35 40 45
   tac tgc aac 153
   Tyr Cys Asn
   50
<210> 4
   <211> 51
   <212> PRT
   <213> Artificial Sequence
<400> 4
   Phe Val Asn Gln His Leu Cys Gly Ser His Leu Val Glu Ala Leu Tyr
   1 5 10 15
   Leu Val Cys Gly Glu Arg Gly Phe Phe Tyr Lys Ala Ala Lys Gly Ile
   20 25 30
   Val Glu Gln Cys Cys Thr Ser Ile Cys Ser Leu Tyr Gln Leu Glu Asn
   35 40 45
   Tyr Cys Asn
   50
<210> 5
   <211> 27
   <212> PRT
   <213> Artificial Sequence
<220>
   <221> MISC_FEATURE
   <222> (1)..(27)
   <223> Insulin B Chain B27K-DTrI
<400> 5
   Phe Val Asn Gln His Leu Cys Gly Ser His Leu Val Glu Ala Leu Tyr
   1 5 10 15
   Leu Val Cys Gly Glu Arg Gly Phe Phe Tyr Lys
   20 25

## Description

### Background

This invention relates to the medical and pharmaceutical fields. More particularly, this invention relates to a novel insulin B chain, a monomeric insulin molecule containing the B chain, pharmaceutical compositions containing the monomeric insulin, the preparations and the applications of the monomeric insulin.

### Background to the Invention

Diabetes is a growing public health threat in the world. Hyperglycemia leads to various diabetes complications that is a major cause of mortality. Tight serum glucose control is the most important factor for preventing and postponing late complications of diabetes, see *The effect of intensive treatment of diabetes on the development and progression of long-term complications in insulin-dependent diabetes mellitus. The Diabetes Control and Complications Trial Research Group.* N Engl. J Med., 1993 **329** (14) p.977-86.

Ever since the discovery of insulin in 1920s it has been used for the treatment of diabetes as an irreplaceable drug. Secretion of insulin in normal human body is regulated by blood glucose concentration. Serum insulin concentration will rise to maximum 30-60 minutes after meal and will recover to base level 4-5 hours later, thus avoid considerable fluctuation of blood glucose concentration caused by food-taking. Regular insulin preparations or administration cannot mimic the process of natural physiological insulin secretion. These therefore can not effectively control rise of serum glucose after meals and there is as a result a risk of hypoglycemia. The reason lies in that insulin exists in form of dimer and hexamer at the concentration of over 100nM in a neutral medium and works by binding to specific receptor after being dissociated into monomer. This association between insulin molecules enhances the stability of insulin and ensures precise and flexible regulation of serum glucose. However, it is an obstacle to absorbance of insulin. Insulin preparation existing in the form of a hexamer will not be absorbed through capillaries into blood until it is transformed to dimeric insulin and subsequently to monomeric insulin after injection through dilution by 50,000 to 100,000 times. The multiple dilution in muscle or subcutaneously is a slow and long process with only 50% insulin being absorbed within 2-4 hours. 90-120 minutes after subcutaneous injection of insulin preparation, the insulin concentration in blood reaches a relatively low peak and fails to decrease rapidly later, see Brange, J. and A. Volund, *Insulin analogs with improved pharmacokinetic profiles.* Adv Drug Deliv. Rev, 1999.**35**(2-3)307-335.

Insulin with low a tendency to self-association in neutral medium is named monomeric insulin. Monomeric insulin can be absorbed rapidly without multiples of dilution and functions within a shorter time after injection, Therefore monomeric insulin has become one of the major targets of developing new generation of drugs for diabetes. Lyspro, monomeric insulin developed by Eli Lilly Co., has proven to be very effective in clinical trials in Europe and America in 1996. It took effect 15 minutes after subcutaneous injection, the concentration reached a maximum in blood one hour later and recovered to normal level 2-4 hours later. Administration of Lyspro 15 minutes after meal is as effective as that of regular insulin 20 minutes before meal. Currently another monomeric insulin Aspart of Novo Co. is in clinical trial, see Vajo, Z. and W.C. Duckworth, *Genetically engineered insulin analogs: diabetes in the new millennium.* Pharmacol. Rev, 2000.**52**(1)1-9.

We have studied a method to prepare monomeric insulin, DPI (despentapeptide insulin) or DTI (destetrapeptide insulin) by secreting monomeric insulin precursor (MIP) out of Saccharomyces *Cerevisiae* and then by enzymatic transpeptidation, see Cui, D. F., Li, M. Y., Zhang, Y.S. and Feng, Y. M. *Monomeric destetrapeptide human insulin from a precursor expressed in Saccharomyces cerevisiae.* J Pept. Res., 2001.**57**(3) 188-92. [as also disclosed in Chinese Patent ZL98 110912.8]. However, the monomeric insulin cannot be produced on large scale because of the following defects in the process: (1) it requires chemical peptide synthesis, for example, GFFY (But) Obut; (2) the yield is below 80% after enzymatic transpeptidation with trypsin; (3) the intermediate after enzymatic transpeptidation requires treatment with strong acid, e.g. TFA, to remove the protecting group; (4) the process is complicated and there exists chemical side reaction.
Consequently, it is an urgent task in this field to develop novel monomeric insulin that can be produced on large scale and by simple preparation.

### Summary of the Invention

One object of this invention is to provide novel monomeric insulin that can be produced on large scale and by simple method.
Another object of this invention is to provide a pharmaceutical composition containing the monomeric insulin.
Moreover, this invention aims to provide the preparation and application of the monomeric insulin and the pharmaceutical composition.
The first aspect of this invention provides insulin B chain comprising the following amino acid sequence:
FVNQH LCGSH LVEAL YLVCG E R X₂₃X₂₄X₂₅X₂₆Y₂₇
wherein, X₂₃, X₂₄, X₂₅ and X₂₆ are individually independent amino acids and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg.

In one preferred embodiment Y27 is Lys.
In another preferred embodiment X23X24X25X26 is GFFY.
The second aspect of this invention provides an insulin molecule with the above B chain.
In one preferred embodiment the insulin is monomeric.

In another preferred embodiment the sequence of the said insulin is: wherein, the amino acids X₂₃, X₂₄, X₂₅ and X₂₆ are individually independent and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg.

The third aspect of this invention provides a pharmaceutical composition containing the monomeric insulin described in this invention and pharmaceutically acceptable carrier.

The fourth aspect of this invention provides a monomeric insulin precursor containing the B chain, a connecting peptide and an insulin A chain from an amino terminal to a carboxyl terminal, wherein the connecting peptide is Ala-Ala-Lys.

The fifth aspect of this invention provides DNA encoding the insulin B chain or the monomeric insulin precursor. It also provides the vector containing the DNA and the host cell containing the DNA or the vector.

The sixth aspect of this invention provides a method to prepare the insulin, in which following steps are included:
a host cell containing a DNA-encoding monomeric insulin precursor is cultured for expression, wherein the said monomeric insulin precursor is expressed, the precursor comprising an insulin B chain, a connecting peptide and an insulin A chain from an amino terminal to a carboxyl terminal and wherein the insulin B chain contains following amino acid sequence:
   F V N Q H L C G S H L V E A L Y L V C G E R X₂₃X₂₄X₂₅X₂₆Y₂₇
   wherein, X₂₃, X₂₄, X₂₅ and X₂₆ are individually independent and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg and the connecting peptide is Ala-Ala-Lys;
Purification of the monomeric insulin precursor;
Enzyme cleavage of the monomeric insulin presucor by trypsin;
Purification of the monomeric insulin.

### Brief description of the drawings

Figure 1 shows the purification of DOI on DEAE-Sephadex A25. The mobile phase has a pH of 7.3, 50mmol/L Tris buffer containing 40% isopropanol; elution gradient 0.05-0.2mol/L NaCl from start to finish. The main peak is DOI. The abscissa is the elution volume and the ordinate is absorbance at 280nm.
Figure 2 shows the thin layer chromatography of Gly-Phe-Phe-Tyr-Ls (Boc) Obut; on the left-hand side crude product; on the right-hand side purified product. The developing solution was a mixture of 3 volumes of solution A (pyridine/acetic acid/water 4:1:1.5 by volume) and 7 volumes of solution B (ethyl acetate/isopropanol 10:4 by volume). The chromatogram was stained by chlorine-starch-KI.
Figure 3 shows a RP-HPLC analysis of Gly-Phe-Tyr-Lys (Boc) Obut, C8 column (Beckman, 4.6×250mm), the mobile phase Solution A was water with 0.1 %TFA and Solution B was 70% acetonitrile with 0.1% TFA. Elution gradient is 0-100% Solution B in (0-50min), flow rate 1 ml/min. The abscissa is the elution time and the ordinate is the absorbance at 280nm.
Figure 4 shows polyacrylamide gel electrophoresis at pH of 8.3 with gel concentration of 15%, stained with Coomassie brilliant blue. From left to right respectively are: DOI, enzymatic reaction solution, B27K-DTrI pure product, porcine insulin.
Figure 5 shows a separation of enzymatic reaction solution on Sephadex G50 (f) column (1.6×60cm), mobile phase is 30% acetic acid, peak 2 is the mixture of B₂₇K-DTrI and DOI. Abscissa is elution volume and the ordinate is absorbance at 280nm.
Figure 6, RP-HPLC separation profile of B₂₇K-DTrI (Boc) Obut, C8 column (Beckman, 10×250mm), the mobile phase Solution A was water with 0.1 %TFA and Solution B was 70% acetonitrile with 0.1% TFA. Elution gradient 10-60% Solution B in (10-40min), flow rate 2ml/min. Peak 1 is DOI, peak 2 is B₂₇K-DTrI (Boc) Obut and peak 3 is Gly-Phe-Phe-Tyr-Ls (Boc) Obut. The abscissa is the elution time and the ordinate is absorbance at 280nm.
Figure 7 shows RP-HPLC separation profile of B₂₇K-DTrI, C8 column (Beckman, 10× 250mm), the mobile phase Solution A was water with 0.1%TFA and Solution B was 70% acetonitrile with 0.1% TFA. Elution gradient 10-60% solution B in (10-40min), flow rate 2ml/min. The main peak is B₂₇K-DTrI. The abscissa is elution time and the ordinate is absorbance at 280nm.
Figure 8 shows a B₂₇K-DTrI HPLC analysis profile, C8 column (Beckman, 4.6x 250mm), and the mobile phase Solution A was water with 0.1 %TFA and Solution B was 70% acetonitrile with 0.1% TFA. Elution gradient 30-70% Solution B in (10-40min), flow rate 1ml/min. The main peak is B₂₇K-DTrI. The abscissa is elution time and the ordinate is absorbance at 230nm.
Figure 9 shows B₂₇K-DTrI electrospray mass spectrographic analysis (ESI-MS), voltage of the electrospray is 4.25KV and capillary temperature is 2000. Theoretical molecular weight is 5508.3 and the actual molecular weight is 5509.0 with the error being 0.01 %.
Figure 10 shows the effect of protein concentration on gel chromatography of zinc free porcine insulin (up) and B₂₇K-DTrI (down), Superdex 75(HR 10/30) column, eluted by pH7.4 phosphate buffered saline, flow rate 0.4ml/min; protein concentrations from low to high respectively are: 40, 80, 200, 400, 600µmol/L. The abscissa is elution time and the ordinate is absorbance at 280nm.

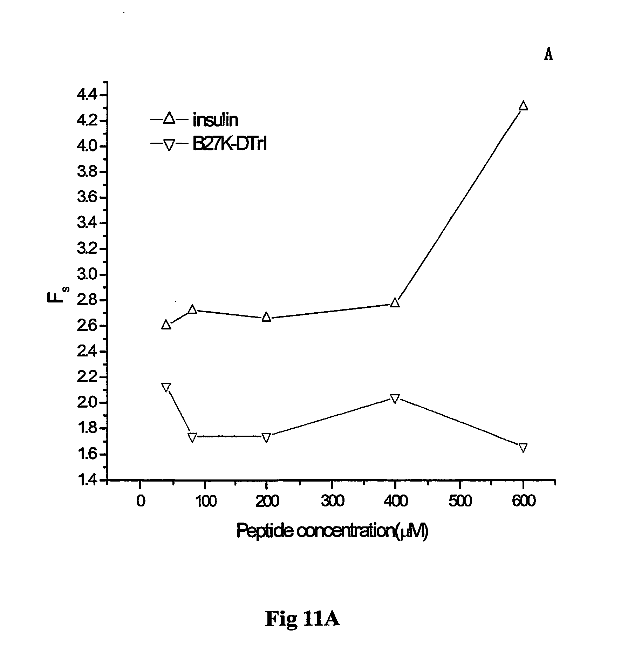

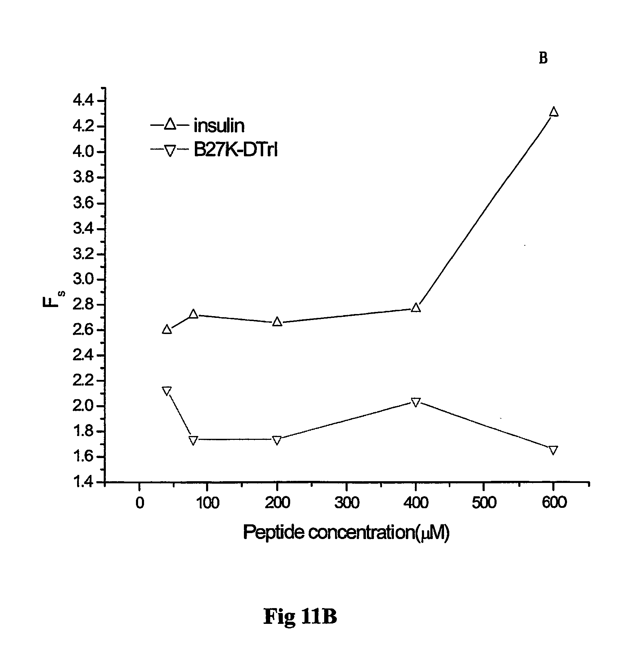

Figure 11 shows the effect of protein concentration on peak shape (up) and retention time (down). The abscissa is the protein concentration, Fs on the ordinate is the symmetric factor and Kav is the retention factor.
Figure 12 shows the gel electrophoresis of zinc free porcine insulin (I), Lyspro (II), B₂₇K-DTrI (III) and DPI (IV) on Superdex 75 (HR 10/30) column, eluted by pH7.4 phosphate buffered saline, flow rate 0.4ml/min; protein concentration is 80µmol/L.
Figure 13 shows the primary structure of a monomeric insulin of this invention.
Figure 14 shows the comparison between the production processes of B₂₇K-DTrI of this invention with that of prior art DTI as taught in China patent ZL98 110912.8.

### Detailed Description

The addition of an alkaline amino acid to the insulin B chain C-terminal would simplify the production process of insulin analogue without affecting its activity. For example, insulin analogue B₂₇K-DTrI, obtained by adding another alkaline amino acid in DTI (destetrapeptide- (B₂₇₋₃₀)-insulin), not only possesses properties of monomeric insulin but has in-vivo bioactivity of 80% of that of native insulin. The process of obtaining B₂₇K-DTrI by enzyme cleavage of monomeric insulin precursor secreted by yeast instead of enzyme transpeptidation using conventional techniques consequently increases total yield and is favorable for industrialized production.
The term "DOI" used in this invention refers to desoctapeptide- (B₂₃₋₃₀)-insulin.
The term "B₂₇K-DTrI" used in this invention refers to destripeptide with amino acids in 27 positions in the B chain being Lys. The base structure is as follows:
F V N Q H L C G S H L V E A L Y L V C G E R X₂₃X₂₄X₂₅X₂₆Y₂₇ (SEQ ID NO:1)
wherein, X₂₃, X₂₄, X₂₅ and X₂₆ are individually independent and are selected from the group of amino acids consisting of Gly, Ala, Asp, Glu, Asn, Gln, Ser, Thr, Leu, Ile, Phe, Tyr, Trp, Pro, Met, His, Val or are not-present (i.e. vacant). At most one not present amino acid among the amino acids X₂₃, X₂₄, X₂₅ and X₂₆ is allowed. Y₂₇ is chosen to be either Lys or Arg. According to the research, the insulin will have certain activity provided it contains the first 25 amino acids (from the 23rd position, amino acid can be optionally changed). In this invention, the amino acid in the 23rd-26th positions can be changed. The changed amino acid preferably is any one of the amino acids except Lys, Arg and Cys, especially L- amino acid.

The peptide of this invention refers to B₂₇K-DtrI, an insulin containing B₂₇K-DtrI and a corresponding monomeric insulin precursor (MIP). The peptide of this invention covers a recombinant peptide, a synthetic peptide or preferably recombinant polypeptide. It can be chemically synthesized or obtained from prokaryotic or eukaryotic hosts (e.g. bacteria, yeast, higher plant, insect and mammalian cells).

The polynucleotide of this invention can be either in DNA or RNA form. The DNA can be single-stranded or double-stranded. The DNA can be a coding strand or not. Full length or fragments of the nucleotide sequence of B₂₇K-DTrI of this invention can be obtained by PCR amplification, recombinant engineering or synthesis. The related sequence can be obtained initially by synthesis. Usually small fragments are synthesized first and then linked to obtain the full length.

Once the sequence is obtained, the sequence can be produced on a large scale by recombinant engineering. Usually the sequence is cloned in a vector, transferred to a host cell and then produced by conventional means. The resulting sequence is purified from the host cell after proliferation.

In this invention, the B₂₇K-DTrI polynucleotide sequence can be inserted into the recombinant expression vector. The term "recombinant expression vector" refers to a bacterial plasmid, a bacteriophage, a yeast plasmid, a plant cell virus, a mammal cell virus such as adenovirus, retrovirus or other vectors that are well known in this field.

In summary, any plasmid and vector that is able to replicate and remain stable can be used. One important characteristic of the expression vector is that it usually contains replication origin, promoter, marker, gene and translation control components.

The expression vector containing the B₂₇K-DTrI coding DNA sequence and appropriate transcription/translation control signals can be constructed by methods familiar to skilled persons in this field. These methods include in-vitro recombination DNA technique, DNA synthesis, in vivo recombination etc. The DNA sequence can be effectively connected to a proper promoter in the expression vector to guide synthesis of mRNA. The typical examples of these promoters are: lac or trp promoter of E.coli., eukaryotic promoter including CMV immediate early promoter, HSV thymidine kinase promoter, early and late SV40 promoter and other promoters expressed in prokaryotic or eukaryotic cells or in viruses by some controllable genes. The expression vector also includes ribosome binding site for translation initiation and transcription terminator.

In addition, the expression vector preferentially contains one or more selective marker genes for providing phenotypic characters for selecting transformed host cells such as dihydrofolate reductase for eukaryotic cell culture, neomycin resistivity and green fluorescin protein (GFP) or tetracin, ampicillin for E.coli.

The vector containing the proper DNA sequence and the promoter or control sequence described above can be used to transform the proper host cell so that it can express protein.

The host cells can be prokaryotic cells, e.g. bacterial cells; or lower eukaryotic cells, e.g. yeast cells; or higher eukaryotic cells, e.g. mammalian cells. The typical examples are: E.coli, Streptomyces; fungal cells, e.g., yeast; plant cells; insect cells of fruit fly S2 or S9; animal cells including CHO, COS etc.

The addition to the host cell of recombinant DNA can be carried out by conventional techniques familiar to person skilled in this field. As the host is a prokaryotic organism such as E.coli, the competent cell that can absorb DNA can be collected after exponential phase of growth and then treated with CaCl₂, steps of which are well known in this field. Another method is to use MgCl₂ instead of CaCL₂. If required, electroporation can also be applied during transformation. As the host is a eukaryotic organism, the following DNA transfection methods can be used: calcium phosphate co precipitation, conventional methods including microinjection, electroporation, liposome entrapment, etc.

The transforming factor thus obtained can be conventionally cultured to express the peptide encoded by the gene of this invention. The culture medium used in the cultivation is selected from various conventional culture media according to the host cell used. The culture is carried out under the conditions suitable for growth of the host cell. As the host cell grows to a certain cell density, a selected promoter is induced by an appropriate method (temperature switching or chemical induction) to culture the cell further.

The recombinant peptide of the methods described above can be expressed in the cell or secreted out of the cell. If required, various separation methods can be used to purify the recombinant protein according to the physical, chemical and other characters of the peptide. These methods are well known in this field. Examples of these methods include but not limited to: conventional repatriation, protein precipitant (salting out), centrifugation, permeation, super centrifugation, size exclusion chromatography (gel electrophoresis), adsorption chromatography, ion exchange chromatography, HPLC and other various liquid phase chromatography techniques and combination thereof.

In one preferred example, B₂₇K-DTrI is firstly expressed in the form of a monomeric insulin precursor and is then obtained after treatment of enzyme cleavage. The monomeric insulin precursor contains the insulin B chain of this invention, a connecting peptide and an insulin A chain from an amino terminal end to a carboxyl terminal end. There is no specific choice of the connecting peptide provided it has a connective function and the amino acid residue connected to A chain is Lys or Arg. An example of one kind of connecting peptide is (Ala)2-5Lys, for example Ala-Ala-Lys.
Any enzyme e.g. trypsin, used to cleave Lys and/or Arg, can be used for enzyme cleavage treatment. Conditions for cleavage change according to the enzyme used. One example of the conditions is: solvent: 0.02-0.05M Tris buffer, pH: 7-8, concentration of substrate at about 5mg/ml, weight of trypsin is about 1/50 of substrate, 4-25 □, 1-6 hour. After enzyme cleaving MIP, the product, B₂₇K-DTrI of this invention, is purified by size exclusion chromatography

This invention also provides a pharmaceutical composition that contains a safe and effective dosage of B₂₇K-DTrI peptide of this invention and a pharmacologically acceptable carrier or excipient. The kind of excipient includes (but not limited to): saline, buffer, glucose, water, glycerol, ethanol and the compound. The drug preparation and the drug delivery method must match. The pharmaceutical composition of this invention can be made into a dosage for injection, for example, prepared in conventional way in physiological saline or aqueous solutions containing glucose and other co-adjuvants. The pharmaceutical composition such as tablet and capsule can also be prepared in a conventional way. All of these forms including the dosage for injection, solution, tablet and capsule will be manufactured under sterile conditions. The delivery quantity of active components is effective quantity for treatment, for example, about 1µg/kg (weight)-about 5mg/kg (weight). The peptide of this invention can be used together with other drugs as well.
The pharmaceutical composition of this invention can be used to treat diabetes and its complications. The safe and effective dosage of B₂₇K-DTrI protein into mammal is usually at least 10µg/kg (weight) and usually not more than 10mg/kg (weight), with the preferable dosage being about 10µg/kg (weight)-100 µ g/kg (weight). Certainly, delivery method, health condition of patient etc. must be taken into consideration when the drug is delivered, which falls upon responsibility of doctors.
Monomeric insulin of this invention can not only be solely used but used together with other drugs (e.g. other insulins) for treating diabetes.

The main advantages of this invention lie in:
The insulin of this invention has a strong monomeric property.
The monomeric insulin of this invention can be obtained by one-step enzyme cleavage from monomeric insulin precursor without enzyme transpeptidation or renaturation.
The pharmaceutical composition containing the insulin has a high bioavailability when delivered not by injection.

The following examples are quoted to further elaborate this invention, but not to limit it. Where no concrete condition is mentioned, normal requirements specified in Molecule Cloning: Laboratory Manual (New York: Cold Spring Harbor Laboratory Press, 2001) by Sambrook et. al. or suggested by the manufacturer shall apply.

### Example 1 Preparation of DOI

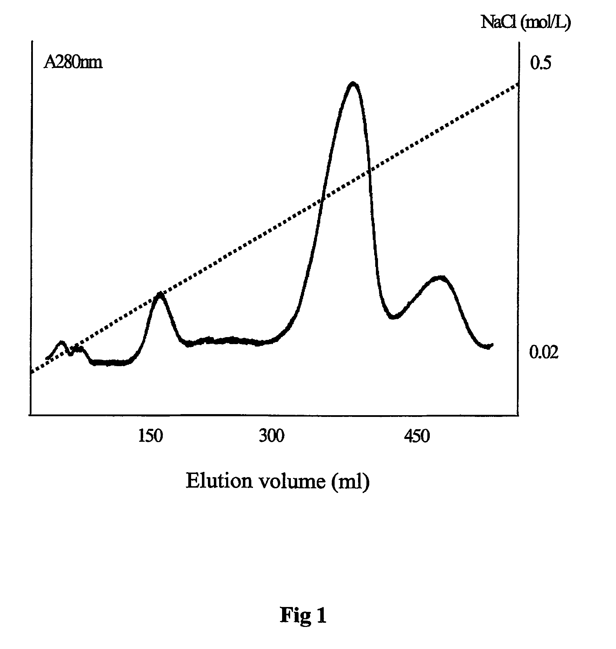

613mg zinc free porcine insulin was dissolved in 127ml 0.05mol/L borax solution (containing 0.001M CaCl₂), pH 8.9. The solution was stirred at 37°C in water bath for 3 hours after which 24.5mg crystal trypsin was added, then the major peak was pooled (Fig.1), dialyzed against water and lyophilized after the solution was purified on DEAE-Sephadex A25 ion exchange column. DOI was thus obtained after desalination on Sephadex G25.

### Example 2 Preparation of GFFYK (Boc) Obut

### 2.1 Preparation of Boc-Phe-Osu

3.18g Boc-Phe (12mmol) and equimolar N-hydroxysuccinimide (HOSu) were mutually dissolved in 15ml THF. The reaction mixture was kept at salt ice bath (-5°C) for 10 minutes and then an equimolar amount of dicyclohexcylcarbodiimide (DCCI) dissolved in 2ml anhydrous tetrahydrofuran (THF) was dripped slowly into the reaction bottle with magnetic stirring at -5°C. After stirring at -5°C more than 2 hours, the solution was allowed to stand overnight at 4°C. The urea which formed was separated by filtration with three-layer filter paper and the filtrate became a white solid. The residue was dissolved with 15ml isopropanol and recrystallized at room temperature to yield 2.5 g (53%) product after washing and drying. The product had a melting point of 138-140°C, was homogeneous in thin layer chromatography, 3/7 system. The developing solution was a mixture of 3 volume of solution A (pyridine/acetic acid/water 4:1:1.5 by volume) and 7 volumes of solution B (ethyl acetate/isopropanol 10:4 by volume). The chromatogram was stained by chlorine-starch-KI.

### 2.2 Preparation of (TFA) Phe-Tyr-OC₂H₅

5.97 g (16.5 mmol) Boc-Phe-Osu and equimolar HCl-Tyr-OC₂H₅ were mutually dissolved in 80 ml anhydrous ethyl acetate and was neutralized with approx. equimolar N-methylmorpholine to pH8. The reaction mixture was stirred overnight at room temperature and washed three times respectively with 10% citric acid, 1% potassium carbonate and saturated sodium chloride. The residue was dried with anhydrous sodium sulfate for 2 hours and again rotary evaporated to an exceedingly small volume. The product was crystallized with petroleum ether, to yield 6.1 g (81 %) after washing and drying. The product had a melting point of 126-128°C, homogeneous in thin layer chromatography (3/7 system). The crystal was dissolved in 50ml dichloromethane (DCM), 25ml trifluoroacetic acid (TFA) was dripped into the solution. After being stirred at room temperature for 35 minutes, the solution was concentrated under vacuum to remove the solvent. The product (TFA) Phe-Tyr-OC₂H₅ was thus obtained after washing more than five times with DCM.

### 2.3 Preparation of (TFA) Phe-Phe-Tyr-OC₂H₅

13 mmol (TFA)-Phe-Tyr-OC₂H₅ was dissolved in 20ml anhydrous THF (solvent) and neutralized to pH7 with 5.4ml N-methylinorpholine. The solution was kept at salt ice bath. Then an equimolar amount of Boc-Phe-OSu was dissolved in 35ml anhydrous THF and was dripped into a (TFA)-Phe-Tyr-OC₂H₅ solution, keeping the reaction solution at neutral pH with N-methylmorpholine. The reaction mixture was stirred overnight at room temperature. After rotary evaporation to powder, the mixture was dissolved with ethyl acetate and washed three times respectively with 10% citric acid, 1% potassium carbonate, saturated sodium chloride. The residue was dried with anhydrous sodium sulfate for 2 hours and again rotary evaporated to an exceedingly small volume. The crude product Boc-Phe-Phe-Tyr-OC₂H₅ was crystallized with a large amount of ether to yield 2g (30%). The product had a melting point of 106-108□, homogeneous in thin layer chromatography (3/7 system). All the product was dissolved in 25ml DCM and 25ml TFA was dripped into the solution. After being stirred at room temperature for 30 minutes, the solution was concentrated under vacuum to remove the solvent. The product (TFA) Phe-Phe-Tyr-OC2H5 was thus obtained after more than five times washing with DCM.

### 2.4 Preparation of Z-Gly-Osu

10g (47mmol) Z-Gly and equimolar amount of Hosu were mutually dissolved in 100ml anhydrous THF (a solvent). The reaction mixture was kept at salt ice bath (-5°C) for 10 minutes and then equimolar amount of DCCI dissolved in 2ml anhydrous THF was dripped slowly into the reaction bottle with magnetic stirring at -5°C. After stirring at -5°C more than 2 hours, the solution was allowed to stand overnight at 4°C. The urea formed was separated by filtration with a three-layer filter and the filtrate became slightly oily after rotary evaporation. The residue was dissolved with a small amount of DCM, then a large amount of ether was added, the filtrate was scrubbed against the bottle wall until yellow precipitate formed and the solution became clear. The clear solution was poured out and allowed to stand at room temperature. The product crystallized and was collected by filtration, yield 6.5g (46%) of product after washing and drying. The product had a melting point of 100-102°C and was homogeneous in thin layer chromatography are (3/7 system).

### 2.5 Preparation of Z-Gly-Phe-Phe-Tyr-OC₂H₅

3.3g mmol (TFA) Phe-Phe-Tyr-OC₂H₅ and 5mmol Z-Gly-Osu was mutually dissolved in 10ml of anhydrous THF (solvent). The solution was neutralized with N-methylmorpholine pH8.0 and then stirred for 72 hours at room temperature. After rotary evaporation to powder, the powder was dissolved in ethyl acetate and washed three times in turn with 10% citric acid, 1% potassium carbonate, saturated sodium chloride. The residue was dried with anhydrous sodium sulfate for 2 hours and again rotary evaporated to an exceedingly small volume. The solution then became muddy from clear then clear again after the addition of a large amount of ether. A grey yellow precipitate formed after standing at room temperature for 72 hours. The precipitate was 2g (87%) after washing with ether and drying, the precipitate had a melting point at 148-151 □.The product was homogeneous in thin layer chromatography (3/7 system).

### 2.6 Preparation of Z-Gly-Phe-Phe-Tyr-NHNH₂

15mmol 85% hydrazine hydrate was added to a solution of 2g (2.9mmol) Z-Gly-Phe-Phe-Tyr-OC₂H₅ in 9ml anhydrous methanol. The reaction mixture was refluxed for 2 hours and then cooled to room temperature. The crystals which formed immediately were collected and washed with 10ml anhydrous ether, 10ml methanol/ether (5v/5v) and water to pH7. The crude yield after drying was 1.29g (65%) and the product was homogeneous in thin layer chromatography (3/7 system).

### 2.7 Preparation of Z-Gly-Phe-Phe-Tyr-Lys (Boc) Obut

37g sodium nitrite was dissolved in 150ml distilled water and 38g ter-butyl alcohol was added to the solution. The reaction mixture was kept in an ice bath for 10 minutes and then 60ml 35% sulfuric acid was dripped slowly into the reaction bottle with magnetic stirring. After stirring in the ice bath more than 30 minutes, the solution was allowed to stand for 15 minutes and then the supernatant was washed three times with 5% sodium bicarbonate. 30ml ter-butyl nitrite was produced after being dried with anhydrous sodium sulfate. 1.25g (1.9mmol) Z-Gly-Phe- Phe-Tyr-NHNH₂ was dissolved in 8ml anhydrous DMF by heating. 2ml 2N HCl/THF (4mmol) was added to the solution and the reaction mixture was kept at -20°C, then 0.3ml ter-butyl nitrite (2.5mmol) was slowly dripped into the solution and shaken for 10 minutes, 10ml anhydrous ethylacetate pre-cooled to -20°C was added to the solution. The mixture was cooled to -30°C. Meanwhile 0.65g (1.9mmol) (HCl)Lys(Boc)Obut was dissolved in 7ml anhydrous DMF, 247µl triethylamine was added into the solution after it was cooled to -30°C, then the solution and 10ml anhydrous ethylacetate pre-cooled to -20°C were successively added into the mixture above. Triethylamine was added to adjust the pH to 8 and then the mixture was allowed to stand at 4°C for 72 hours after being shaken for 30 minutes at 4°C. After rotary evaporation to powder, the residue was dissolved with ethylacetate, then washed three times in turn with 10% citric acid, 1% potassium carbonate, and saturated sodium chloride. The residue was dried with anhydrous sodium sulfate for 2 hours and again rotary evaporated to oil. The product was crystallized with triple times volume of ether, yield 1 g (56%) after washing with cool ethylacetate, cool ether and drying. The product had a melting point of 160-162°C and was homogeneous in thin layer chromatography (3/7 system).

### 2.8 Preparation of Gly-Phe-Phe-Tyr-Lys(Boc)Obut

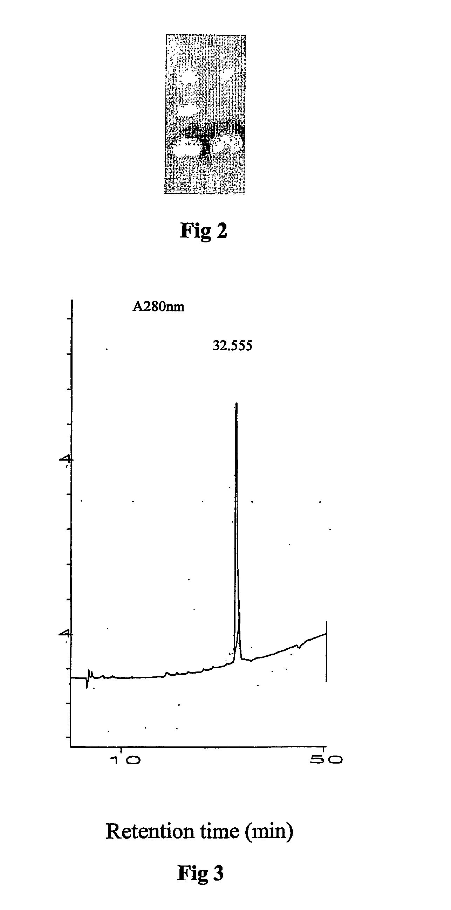

0.56g Z-Gly-Phe-Phe-Tyr-Lys(Boc)Obut was dissolved in 90ml anhydrous methanol and the solution was adjusted to pH 3.0 with glacial acetic acid. 0.12g Pd carbon was added. The reaction solution was stirred and mixed with hydrogen for 6 hours. Then the solution was filtered with diatomite. (Pd carbon was discarded after immersion in chloroform) and the filtrate was dried with rotary evaporation. The product (yield 93%) was obtained after repeatedly washing with ethylacetate, homogeneous in thin layer chromatography (3/7 system) and can be stained with ninhydrin. As shown in Figure 2. HPLC was homogeneous as shown in Fig.3. The analytic value of amino acids was corresponding to that of theoretical value: Glyl.2 (1.0); Tyr1.0 (1.0); Phe2.2 (2.0); Lys1 (1.0).

### Example 3. Preparation of B₂₇K-DTrI

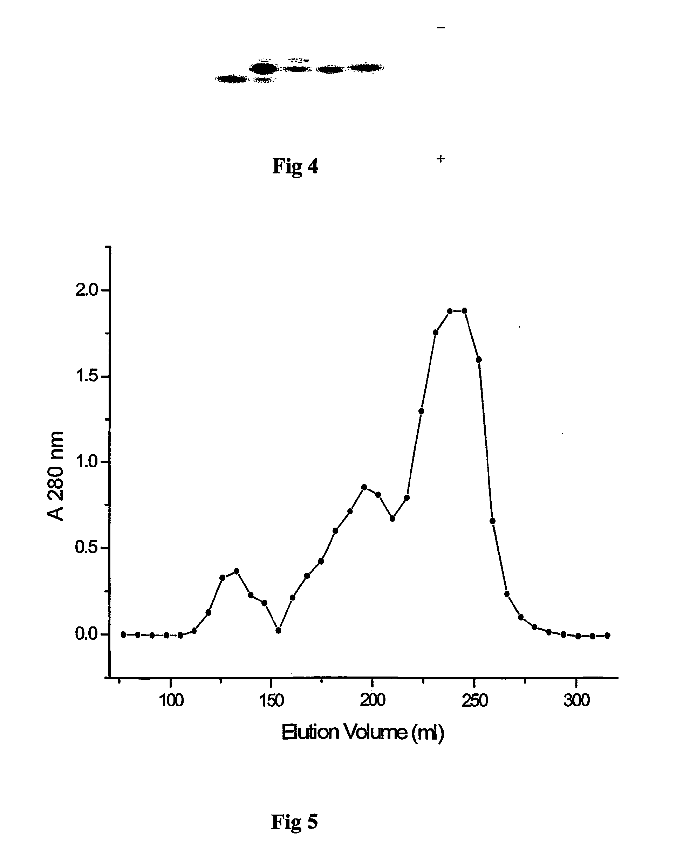

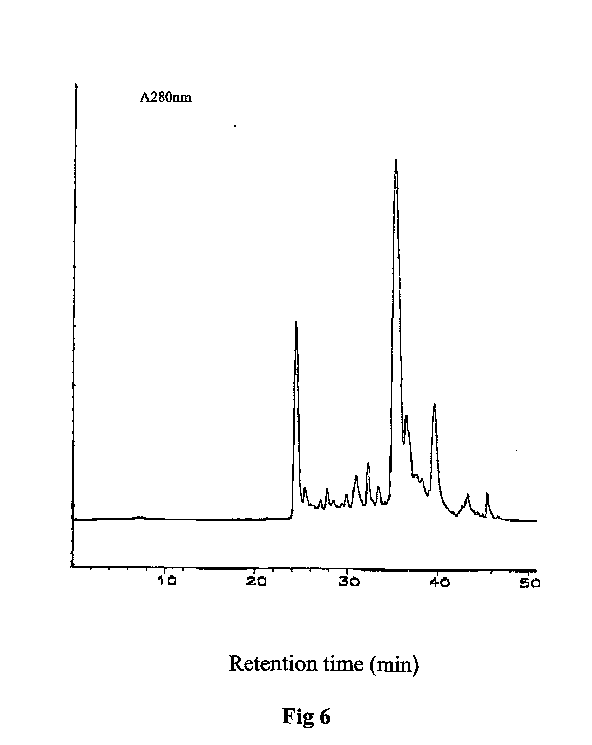

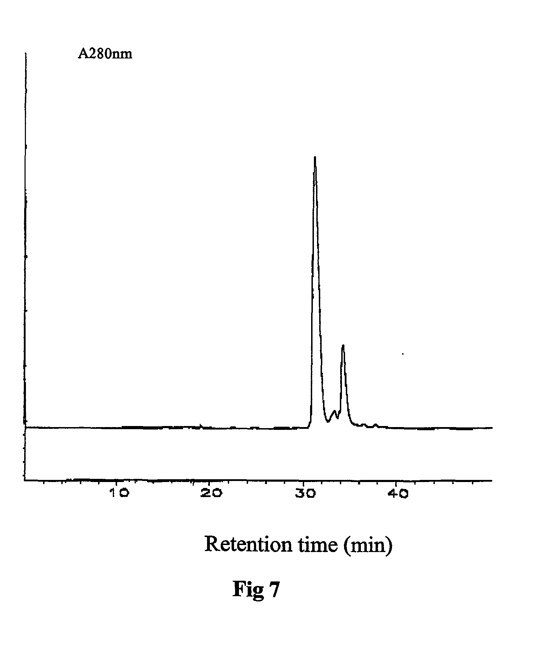

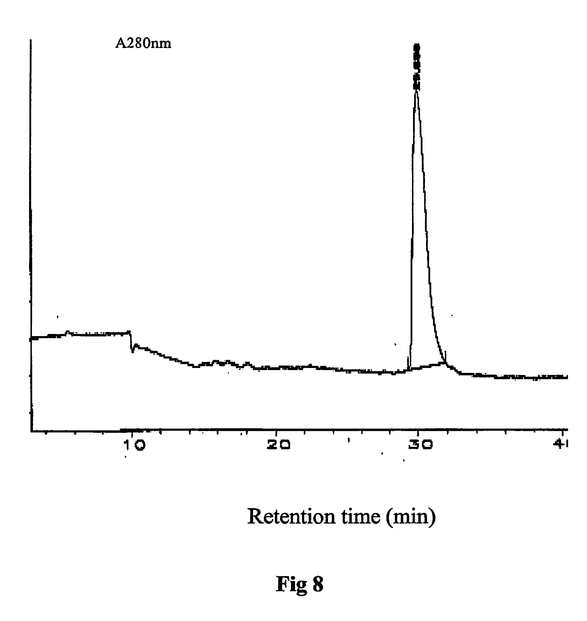

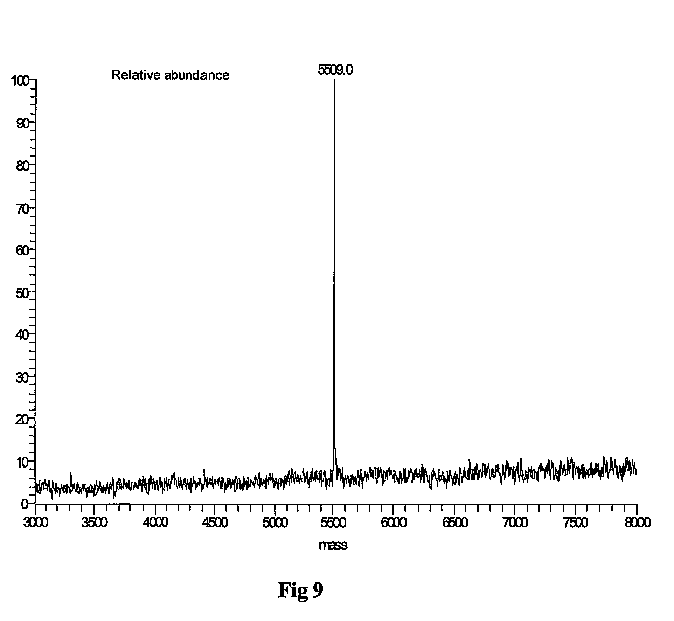

0.237g (0.172mmol) Gly-Phe-Phe-Tyr-Lys(Boc)Obut was dissolved in 0.26ml dimethylsulfone (DMSO) by warming to 50□. Then the solution was incubated at 37°C water bath and 86mg (0.0172mmol) DOI was slowly added to the solution. 1.82ml 1-4 butanediol and 0.52ml water pre-heated to 37°C was added to the reaction solution and the pH was adjusted to 6.5 with 5 µl N-methylmorpholine. 7.8mg TPCK-Trypsin was added and reaction mixture incubated at 30°C. After 2 and 4 hours, 4.5mg TPCK-Trypsin was added each time. After 20 hours, 1.1ml glacial acetic acid was added and the reaction was ended by adjusting the pH to 3 with 1 mol/l HCl. The reaction mixture was purified by Sephadex G50 fine column chromatography with 30% acetic acid as eluent. 76mg (80% yield) crude product of B₂₇K-DTrI (Boc) Obut was obtained (Fig.5). The crude product was purified by C8 RP-HPLC (Fig.6), washed with cold acetone and dried. The dried product was dissolved in anhydrous TFA to the concentration of 3mg/ml. After standing at 10°C for 1 hour, the TFA was evacuated and the residue was washed thoroughly with dichloromethane. The product was purified by C₈ RP-HPLC to obtain B₂₇K-DTrI (Fig.7). The sample was homogeneous in pH 8.3 PAGE (Fig.4) and C₁₈HPLC (Fig.8). The molecular weight was determined by electrospray mass spectroscopy to be 5509Da (Fig.9) and differed from the theoretical molecular weight of 5508.3 by an error of 0.01%. The sequence of 14 N-terminal residues and 2 C-terminal residues were determined to be correct (Table 1). These data indicates that the charge, hydrophilic and hydrophobic properties of the sample are all homogeneous and protein sequence is consistent to the designed sequence

**Table 1 N Terminal and C Terminal Sequences of B₂₇K DtrI (N Terminal→ C Terminal)**

| | |
|---|---|
| N terminal: A chain: | GIVEQCCTSICSLY---- □tested□ |
| | GIVEQCCTSICSLY--- □Theoretical□ |
| B chain: | FVNQHLCGSHLVEA---□tested□ |
| | FVNQHLCGSHLVEA----(Theoretical□ |
| C terminal: A chain:- | --------------N□tested□ |
| | ----------------- N□Theoretical□ |
| B chain: | ---------------- YK□tested□ |
| | ---------------- YK□Theoretical□ |

### Example 4. Determination of Self-association Property

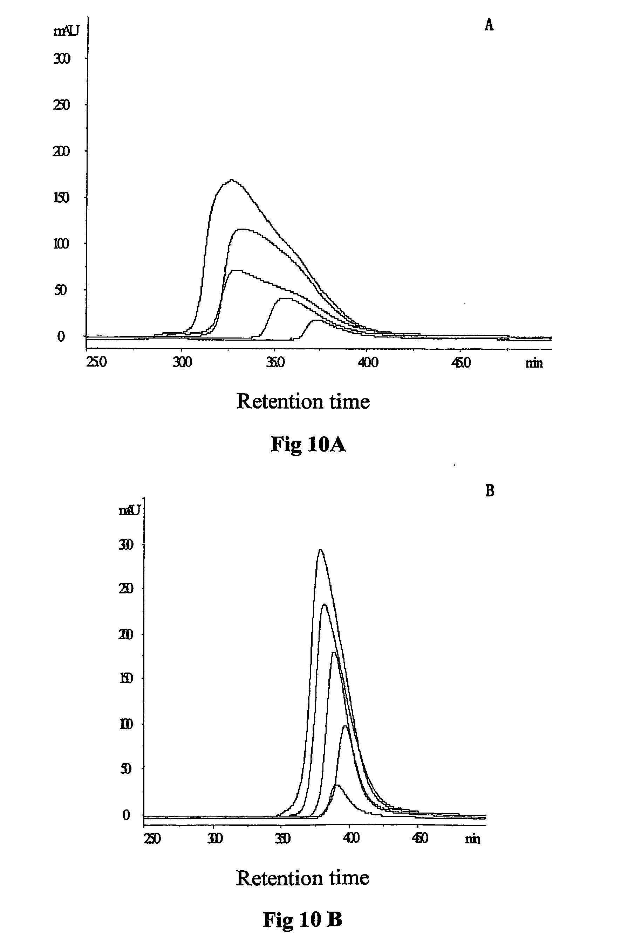

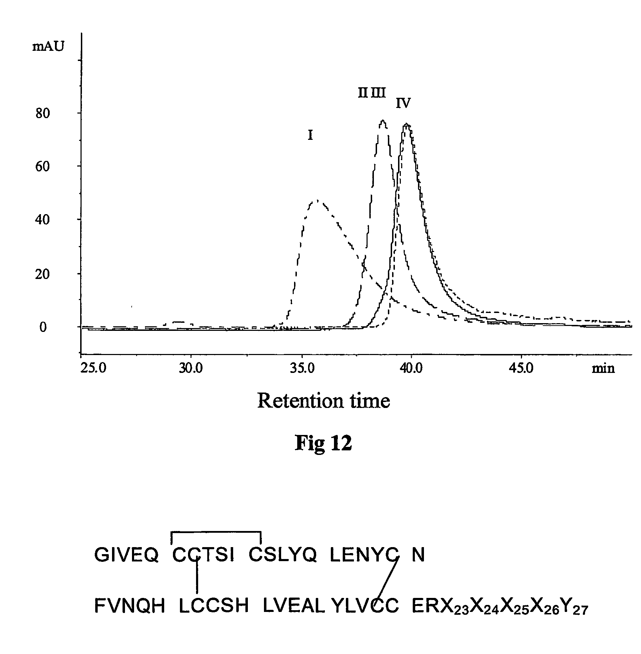

The monomeric behavior of B₂₇K-DTrI was determined by size exclusion chromatography using a Superdex 75 (HR 10/30) column, eluted by PBS (phosphate-buffered saline, pH7.4), with a flow rate 0.4ml/min, loading volume 0.1ml, detected at 280nm, at room temperature. The molecular homogeneity was measured by Symmetry factor Fs. Fs=W0.05h/2A, wherein, W0.05 is the band width at 0.05 peak height and A is the width of the first half peak at 0.05 peak height. The change of average molecular weight was measured by plotting the distribution coefficient Kav vs. protein concentration. Kav=(Vr-V0)/(Vc-Vo), wherein Vr is the retention volume, Vo the void volume and Vc the total bed volume. The retention time on the Superdex 75 column decreased with increasing concentration of zinc free insulin control as shown in Fig. 10A; The retention time of B₂₇K-DTrI was independent of protein concentrations as shown in Fig. 10B. The Fs value of insulin increased markedly at concentrations above 400 umol/L (Fig. 10C) and the Kav value of the insulin decreased with increasing concentration (Fig. 10D). While retention time and peak shape of B₂₇K-DTrI did not change with increasing protein concentration. The monomeric behaviours of Lyspro (B28Lys, B29Pro insulin), DPI (despentapeptide insulin) and B₂₇K-DTrI were compared at the same concentration (80 umol/1) on Superdex 75 column. The Kav values of B₂₇K-DTrI and Lyspro insulin were 0.51 and 0.48 respectively (Fig. 12).

### Example 5 Biological Activity Test

### 5.1 Mouse convulsion test (Chinese Pharmacopoeia, 1985, Appendix 100 pages)

Male Kunmin mice (body weight 27-30g) were randomly divided into four groups with 24 in each group, fasting 2 hours before the experiment. The sample was dissolved in saline, pH 5.0, adjusted with hydrochloric acid, 10D280nm=1mg/L. The potency of the sample was estimated to be 80% of that of standard product, high dosage was 0.09U/ml, low dosage was 0.045U/ml and the solvent was the psychological saline with the pH adjusted to 2.5 by hydrochloric acid. 0.3ml was subcutaneously administered to each mouse within 15 minutes at 25°C and then these mice were kept at 37°C for 90 minutes' observation―all of those that were convulsive, dead and could not turn over when made to lie supine are considered to be positive reaction. The data was determined by qualitative reaction test.

### 5.2 Mouse plasma glucose lowering test (Chinese Pharmacopoeia, 2000, Appendix 106 pages)

Male Kunmin mice (body weight 28-30g) were randomly divided into four groups with 10 in each group. The potency of the sample was estimated to be 80% of that of standard product, high dosage was 0.07U/ml, low dosage was 0.035U/ml and the solvent was the psychological saline with the pH adjusted to 2.5 by hydrochloric acid. 0.25ml sample was subcutaneously administered to each mouse at 25□ and blood from the eye venous plexus was sampled to determine the blood sugar 40 minutes after the injection; the drug was administered to each mouse for the second time three hours later according to double-crossing (high dosage of the sample in test was administered to the group with low dosage of standard product earlier) and the blood sugar was tested after 40 minutes. The data were determined by a quantitative reaction test. The biological activity according to mouse convulsion test is 21U/mg and FL% is 23.5% (<30%) (Table 2); the biological activity according to mouse plasma glucose lowering test is 23U/mg and FL% is 11.9% (<25%) (Table 3); The standard product used in the experiment is 27U/mg insulin provided by NICPBP (The National Institute for the Control of Pharmaceutical and Biological Products). The results from the two testing methods are consistent to each other, and biological activity of B₂₇K-DTrI is 80% of that of native insulin.

**Table 2 B₂₇K-DTrI Biological Activity by Mouse Convulsion Test (Chinese Pharmacopoeia, 1985)**

| Sample | Dosage (U/ml) | Mice Tested | Convulsive Mice |
|---|---|---|---|
| Native Insulin | 0.09 | 24 | 17 |
| | 0.045 | 24 | 4 |
| | | | |
| B₂₇K-DTrI | 0.09 | 24 | 19 |
| | 0.045 | 24 | 2 |

**Table 2 B₂₇K-DTrI Biological Activity by Mouse Plasma Glucose Lowering Test (Chinese Pharmacopoeia, 2000)**

| No. | Group 1 | Group 2 | Group 3 | Group 4 |
|---|---|---|---|---|
| 1 | 69.9 | 28.9 | 61.3 | 31.7 |
| 2 | 68.9 | 42.4 | 50.6 | 35.8 |
| 3 | 64.1 | 42.4 | 53.7 | 51.3 |
| 4 | 64.1 | 24.5 | 65.8 | 48.6 |
| 5 | 65.5 | 54.4 | 66.8 | 39.6 |
| 6 | 62.7 | 32.4 | 55.8 | 47.2 |
| 7 | 64.1 | 58.6 | 54.8 | 25.8 |
| 8 | 68.2 | 40.3 | 55.8 | 40.0 |
| 9 | 61.3 | 42.4 | 48.6 | 40.0 |
| 10 | 52.7 | 57.9 | 54.8 | 40.0 |

| Average Value | 64.1 | 42.4 | 55.8 | 40.0 |
|---|---|---|---|---|
| 1 | 36.9 | 53.4 | 37.2 | 35.8 |
| 2 | 44.1 | 52.4 | 39.3 | 32.7 |
| 3 | 41.3 | 58.6 | 33.8 | 58.2 |
| 4 | 28.9 | 53.4 | 44.1 | 48.2 |
| 5 | 34.1 | 52.0 | 39.3 | 47.2 |
| 6 | 43.4 | 51.7 | 40.6 | 47.2 |
| 7 | 36.9 | 59.9 | 40.0 | 47.2 |
| 8 | 37.9 | 53.4 | 40.6 | 48.9 |
| 9 | 36.9 | 47.9 | 39.3 | 47.5 |
| 10 | 28.9 | 50.6 | 39.3 | 59.6 |
| Average Value | 36.9 | 53.3 | 39.3 | 47.3 |

### Example 6 B₂₇K-DTrI Expressed by Genetic Engineering

In this experiment, MIP (monomeric insulin precursor) was constructed by connecting the C terminal and the N terminal with connecting peptide (e.g. Ala-Ala-Lys, for MIP structure is helpful for insulin folding and formation of correct disulfide bond after biosynthesis. The steps are as follows:
Signal peptide sequence (α-MFL, α-mating factor leader) was added to 5'terminal of MIP gene (see SEQ ID NO:3 below) obtained from chemical synthesis, then the gene was cloned into pPIC9K plasmid to get pPIC9K/MIP by use of enzymatic cleavage site of EcoRI and NotI. pPIC9K/MIP was then transferred into yeast P.pastoris after treatment with linearization and spot hybridization was employed to screen high-copy strain.
SEQ ID No:3:

After high-density fermentation, MIP was obtained from the product by hydrophobic chromatography. The amino acid sequence was shown in SEQ ID NO:4:

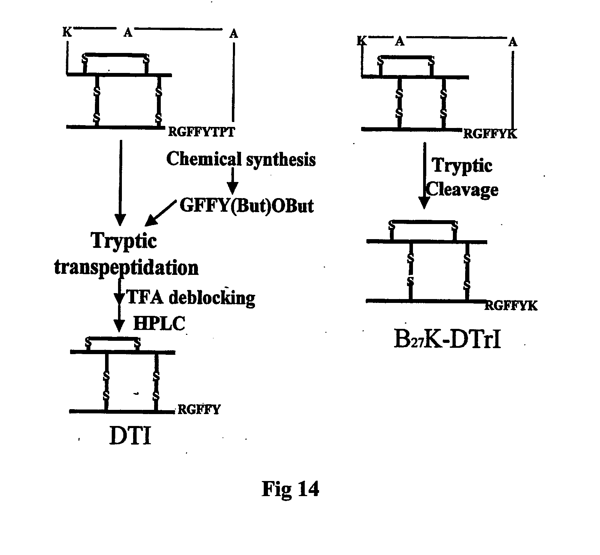

Highly pure B27K-DTrI (structure in Fig. 13) was obtained by enzymatic cleaving MIP with trypsin (solvent: 0.03M Tris buffer, pH: 7.5, 20□, 2 hours) and then by size exclusion chromatography.

### Discussion

B27K-DTrI can also be expressed by other yeast expression system or secretion E.coli expression system with the same technical process. This technical process compared with preparation of DTI disclosed in Chinese patent application 98 1 10912.8 overleaps overelaborate steps of chemical synthesis of peptide GFFY(But)OBut, enzymatic transpeptidation, HPLC separation etc. in conventional technique, consequently increases yield by a big margin and is more suitable for industrialized production. The comparison between the two processes is shown in Fig. 14.

## Claims

1. An insulin B chain comprising the following amino acid sequence:
F V N Q H L C G S H L V E A L Y L V C G E R X₂₃X₂₄X₂₅X₂₆Y₂₇
wherein, X₂₃, X₂₄, X₂₅ and X₂₆ are individually independent amino acids and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg.

2. The insulin B chain according to claim 1 in which Y₂₇ is Lys.

3. The insulin B chain according to claim 1 or claim 2 in which the sequence X₂₃X₂₄X₂₅X₂₆ is GFFY.

4. An destetrapeptide insulin molecule comprising the insulin B chain of one of claims 1 to 3.

5. The destetrapeptide insulin molecule according to claim 4, wherein the insulin is in monomeric form.

6. The insulin molecule according to claim 4 or claim 5 having a structure: wherein, the amino acids X₂₃, X₂₄, X₂₅ and X₂₆ are individually independent and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg.

7. A pharmaceutical composition comprising the destetrapeptide insulin according to one of claims 4 to 6 and a pharmaceutically acceptable carrier.

8. A monomeric insulin precursor comprising the insulin B chain according to claim 1, a connecting peptide and an insulin A chain from an amino terminal to a carboxyl terminal.

9. A DNA sequence encoding the insulin B chain according to claim 1 or the monomeric insulin precursor according to claim 8.

10. An expression vector containing the DNA according to claim 9.

11. A host cell containing the expression vector according to claim 10 or the DNA according to claim 9.

12. A method to prepare insulin comprising the following steps:
a host cell containing a DNA-encoding monomeric insulin precursor is cultured for expression, wherein the said monomeric insulin precursor is expressed, the precursor comprising an insulin B chain, a connecting peptide and an insulin A chain from an amino terminal to a carboxyl terminal and wherein the insulin B chain contains following amino acid sequence:
F V N Q H L C G S H L V E AL Y L V C G E R X₂₃X₂₄X₂₅X₂₆Y₂₇
wherein, X₂₃, X₂₄, X₂₅ and X₂₆ are individually independent and are selected from the group of amino acids consisting of Gly, Ala, Asp, Glu, Asn, Gln, Ser, Thr, Leu, Ile, Phe, Tyr, Trp, Pro, Met, His, Val or are not present, and wherein at most one of the individually independent amino acids is not present; and furthermore wherein Y₂₇ is either Lys or Arg and the connecting peptide is Ala-Ala-Lys;
Purification of the monomeric insulin precursor;
Enzyme cleavage of the monomeric insulin presucor by trypsin;
Purification of the monomeric insulin.

## Patentansprüche

1. Insulin-B-Kette, umfassend die folgende Aminosäuresequenz:
FVNQH LCGSH LVEAL L Y L V C G E R X₂₃ X₂₄ X₂₅ X₂₆ Y₂₇
wobei X₂₃, X₂₄, X₂₅ und X₂₆ individuell unabhängige Aminosäuren sind und ausgewählt sind aus der Gruppe von Aminosäuren bestehend aus Gly, Ala, Asp, Glu, Asn, Gln, Ser, Thr, Leu, Ile, Phe, Tyr, Trp, Pro, Met, His, Val, oder nicht vorhanden sind, und wobei höchstens eine der individuell unabhängigen Aminosäuren nicht vorhanden ist; und ferner, wobei Y₂₇ entweder Lys oder Arg ist.

2. Insulin-B-Kette nach Anspruch 1, in welcher Y₂₇ Lys ist.

3. Insulin-B-Kette nach Anspruch 1 oder Anspruch 2, in welcher die Sequenz X₂₃X₂₄X₂₅X₂₆ GFFY ist.

4. Destetrapeptid-Insulinmolekül, umfassend die Insulin-B-Kette nach einem der Ansprüche 1 bis 3.

5. Destetrapeptid-Insulinmolekül nach Anspruch 4, bei welchem das Insulin in monomerer Form vorliegt.

6. Insulinmolekül nach Anspruch 4 oder 5 mit der Struktur: wobei die Aminosäuren X₂₃, X₂₄, X₂₅ und X₂₆ individuell unabhängig sind und ausgewählt sind aus der Gruppe von Aminosäuren bestehend aus Gly, Ala, Asp, Glu, Asn, Gln, Ser, Thr, Leu, Ile, Phe, Tyr, Trp, Pro, Met, His, Val, oder nicht vorhanden sind, und wobei höchstens eine der individuell unabhängigen Aminosäuren nicht vorhanden ist; und ferner, wobei Y₂₇ entweder Lys oder Arg ist.

7. Pharmazeutische Zusammensetzung, umfassend das Destetrapeptid-Insulin nach einem der Ansprüche 4 bis 6 und cinen pharmazeutisch akzeptablen Träger.

8. Monomerer Insulinprecursor, umfassend die Insulin-B-Kette nach Anspruch 1, ein verbindendes Peptid und eine Insulin-A-Kette von einem Aminoterminal zu einem Carboxylterminal.

9. DNA-Sequenz, kodierend die Insulin-B-Kette nach Anspruch 1 oder den monomeren Insulinprecursor nach Anspruch 8.

10. Expressionsvektor, enthaltend die DNA nach Anspruch 9.

11. Wirtszelle, enthaltend den Expressionsvektor nach Anspruch 10 oder die DNA nach Anspruch 9.

12. Verfahren zum Herstellen von Insulin, umfassend die folgenden Schritte:
eine Wirtszelle, enthaltend einen DNA-kodierenden monomeren Insulinprecursor, wird für die Expression kultiviert, wobei der monomere Insulinprecursor exprimiert wird, wobei der Precursor eine lnsulin-B-Kette, ein verbindendes Peptid und eine Insulin-A-Kette von einem Aminoterminal zu einem Carboxylterminal umfasst, und wobei die Insulin-B-Kette die folgende Aminosäuresequenz enthält.
F V N Q H L C G S H L V E A L Y L V C G E R X₂₃ X₂₄ X₂₅ X₂₆ Y₂₇
wobei X₂₃, X₂₄. X₂₅ und X₂₆ individuell unabhängig sind und ausgewählt sind aus der Gruppe von Aminosäuren, bestehend aus Gly, Ala, Asp, Glu, Asn, Gln, Ser, Thr, Leu, Ile, Phe, Tyr, Trp, Pro, Met, His, Val, oder nicht vorhanden sind, und wobei mit höchstens eine der individuell unabhängigen Aminosäuren nicht vorhanden ist; und ferner wobei Y₂₇ entweder Lys oder Arg ist und das verbindende Peptid Ala-Ala-Lys ist;
Reinigung des monomeren Insulinprecursors;
Enzymspaltung des monomeren Insulinprecursors mittels Trypsin;
Reinigung des monomeren Insulins.

## Revendications

1. Chaîne B de l'insuline comprenant la séquence d'acides aminés suivante :
F V N Q H L C G S H L V E A L Y L V C G E R X₂₃X₂₄X₂₅X₂₆Y₂₇
dans laquelle, X₂₃, X₂₄, X₂₅ et X₂₆ sont des acides aminés individuellement indépendants et sont choisis dans le groupe d'acides aminés constitué par Gly, Ala, Asp, Glu, Asn, Gln, Ser, Thr, Leu, Ile, Phe, Tyr, Trp, Pro, Met, His, val ou ne sont pas présents, et dans laquelle au plus un des acides aminés individuellement indépendants n'est pas présent ; et en outre, dans laquelle Y₂₇ est Lys ou Arg.

2. Chaîne B de l'insuline selon la revendication 1, dans laquelle Y₂₇ est Lys.

3. Chaîne B de l'insuline selon la revendication 1 ou la revendication 2, dans laquelle la séquence X₂₃X₂₄X₂₅X₂₆ est GFFY.

4. Molécule d'insuline des-tétrapeptide comprenant la chaîne B de l'insuline selon l'une des revendications 1 à 3.

5. Molécule d'insuline des-tétrapeptide selon la revendication 4, dans laquelle l'insuline est sous une forme monomère.

6. Molécule d'insuline selon la revendication 4 ou la revendication 5, ayant une structure : dans laquelle, les acides aminés X₂₃, X₂₄, X₂₅ et X₂₆ sont individuellement indépendants et sont choisis dans le groupe d'acides aminés constitué par Gly, Ala, Asp, Glu, Asn, Gln, Ser, Thr, Leu, Ile, Phe, Tyr, Trp, Pro, Met, His, Val ou ne sont pas présents, et dans laquelle au plus un des acides aminés individuellement indépendants n'est pas présent ; et en outre dans laquelle Y₂₇ est Lys ou Arg.

7. Composition pharmaceutique comprenant l'insuline des-tétrapeptide selon l'une des revendications 4 à 6 et un vecteur pharmaceutiquement acceptable.

8. Précurseur d'insuline monomère comprenant la chaîne B de l'insuline selon la revendication 1, un peptide de connexion et une chaîne A de l'insuline d'une extrémité amino terminale à une extrémité carboxyle terminale.

9. Séquence d'ADN codant pour la chaîne B de l'insuline selon la revendication 1 ou le précurseur d'insuline monomère selon la revendication 8.

10. Vecteur d'expression contenant l'ADN selon la revendication 9.

11. Cellule hôte contenant le vecteur d'expression selon la revendication 10 ou l'ADN selon la revendication 9.

12. Procédé de préparation d'insuline comprenant les étapes suivantes :
la culture d'une cellule hôte contenant un ADN codant pour le précurseur d'insuline monomère pour expression, dans lequel ledit précurseur d'insuline monomère est exprimé, le précurseur comprenant une chaîne B de l'insuline, un peptide de connexion et une chaîne A de l'insuline d'une extrémité amino terminale à une extrémité carboxyle terminale et dans lequel la chaîne B de l'insuline contient la séquence d'acides aminés suivante :
F V N Q H L C G S H L V E A L Y L V C G E R X₂₃X₂₄X₂₅X₂₆Y₂₇
dans laquelle, X₂₃, X₂₄, X₂₅ et X₂₆ sont individuellement indépendants et sont choisis dans le groupe d'acides aminés constitué par Gly, Ala, Asp, Glu, Asn, Gln, Ser, Thr, Leu, Ile, Phe, Tyr, Trp, Pro, Met, His, Val ou ne sont pas présents, et dans laquelle au plus un des acides aminés individuellement indépendants n'est pas présent ; et en outre dans laquelle Y₂₇ est Lys ou Arg et le peptide de connexion est Ala-Ala-Lys ;
la purification du précurseur d'insuline monomère ;
le clivage enzymatique du précurseur d'insuline monomère par la trypsine ;
la purification de l'insuline monomère.
